# EUROPEAN PATENT SPECIFICATION

(11) **EP 4 324 434 B1**
(45) Date of publication and mention of the grant of the patent: **24.12.2025**
(21) Application number: 22195474.6
(22) Date of filing: 13.09.2022
(51) Int. Cl.: A61F 5/01, A61H 1/02, A61H 3/00, B25J 9/00

(54) **EXOSKELETON AND HIP JOINT**
EXOSKELETT UND HÜFTGELENK
EXOSQUELETTE ET JOINT DE HANCHE

(30) Priority: 17.08.2022 GB 202212021
(43) Date of publication of application: 21.02.2024
(73) Proprietor: Xothotics Limited, Colyford, Colyton EX24 6TZ (GB)
(72) Inventor: FRYER, Paul, Colyton, EX24 6TZ (GB)
(74) Representative: White, Andrew John

(56) References cited:
- CN-A- 111 991 131
- US-B1- 9 808 073

## Description

### Field of the invention

The present disclosure relates to an exoskeleton for providing support to a human user, in particular a loadbearing exoskeleton for providing support to a user with a lower body resection, such as a lower body amputation or pelvic resection.

### Background

Existing lower body braces, orthoses and prosthetic limbs frequently rely on a load bearing region of the skeletal structure being intact. Load is then transferred or shared between the existing skeletal structure and brace, orthosis, or prosthetic limb.

This is a problem for individuals following a pelvic resection or internal hemipelvectomy as the skeletal structure of the lower body that remains may be incapable of load bearing for the ipsilateral limb. As a result, traditional leg braces or orthoses are often unsuitable as they rely on the pelvis being intact and load bearing. This also often results in overloading of the contralateral limb, causing the individual to tire of standing quickly.

This is also a problem for individuals with lower limb amputations as the residual bone at the interface between the stump and the prosthesis may be subjected to high loading forces. This may be uncomfortable and cause the individual to tire of standing quickly. The loading forces can also injure the skin covering the stump. Following injury, the amputee may be unable to use their prosthesis whilst the skin heals. This can be a particular problem for diabetic amputees.

US 9808073 discloses an exoskeleton system including a hip joint to provide anatomically emulating three degrees of freedom of movement of the hip joint at a hip location of a user, a knee joint to provide anatomically emulating forward and backward movement of the knee joint at a knee location of the user, an ankle joint to provide anatomically emulating forward and backward movement of the ankle joint at an ankle location of the user, a knee rocker arm as part of the knee joint to transfer a load to a ground surface when the knee rocker arm is in contact with the ground surface as manually powered by the user, and a ground rocker arm as part of the ankle joint to transfer the load to a ground surface when the ground rocker arm is in contact with the ground surface as manually powered by the user.

CN 111991131 discloses a joint traction orthosis. The joint traction orthosis comprises a first arm part, a second arm part and a force providing part. The first arm part is connected with the second arm part through the force providing part, when the first arm part rotates relative to the force providing part, the force providing part can provide resistance, and when the second arm part rotates relative to the force providing part, the force providing part can provide the resistance.

### Summary of the invention

The invention is set out in the appended claim 1 as well as in independent claim 9. Optional features are set out in the dependent claims.

According to the invention there is provided an exoskeleton for providing support to a human user. The exoskeleton comprises a torso portion and leg portion, wherein the torso portion and leg portion are coupled via a hip joint. The hip joint comprises a torso arm for detachably coupling to the torso portion and leg arm for detachably coupling to a leg portion, wherein the torso arm and leg arm are both coupled to a central portion of the hip joint via respective rotation limiting joints. The exoskeleton may provide support to a human user by providing a load bearing structure configured to capture at least a portion of the vertical load of the user. This may extend the period of time the user can stand and/or walk without tiring.

The rotation limiting joint for the torso arm has a first range of motion about the central portion, and the rotation limiting joint for the leg arm has a second range of motion about the central portion. The second range of motion may be less than the first range of motion. The hip joint may be advantageous to allow movement of the leg portion relative to the torso portion, facilitating movement and flexibility of the user of the exoskeleton. The rotation limiting joint may also be advantageous to prevent over-rotation / hyperextension of the leg portion relative to the torso portion, and vice versa. This may provide support to the user in use and prevent collapse of the exoskeleton. The rotational limiting joint may allow for a normal human range of movement, advantageously allowing the leg portion to move relative to the torso portion whilst seated, standing, and during walking.

The hip joint may be configured to operate in a parasagittal plane of the human body.

The exoskeleton may further comprise a buttock support arm coupled to a buttock support portion. The buttock support arm may be coupled to the hip joint via a buttock rotation limiting joint, wherein the buttock support arm has a third range of motion. In some examples, the buttock support portion is directly coupled to the central portion of the hip joint, wherein the central portion is configured to rotate.

The buttock support portion may be configured to encompass the lower and mid buttock from the midpoint of the back at the gluteal sulcus to the upper front of the thigh at the top of the rectus femoris muscle with the lower edge following the line of the gluteal sulcus.

The buttock support may be advantageous for load capture, in particular vertical load capture. This may reduce or eliminate load capture at the base of the axilla of the user (under the armpits) which can be uncomfortable and damaging to the axillary nerve of the user.

In some examples, the buttock support portion may further comprise an inflatable means, such as an inflatable bag or cushion. The inflatable portion may be configured to inflate to fill void space between the buttock support portion and the buttock of the user. This may be advantageous to improve the loading interface between the user and the buttock support portion. This may also improve the comfort of the buttock support portion in use.

The torso arm, the leg arm and the buttock support arm may be configured to rotate about a common axis of the hip joint.

The leg portion may further comprise an upper leg brace, for example a thigh brace, and a lower leg brace, for example a calf brace. The upper leg brace and the lower leg brace may be coupled by a knee joint. This may be advantageous to allow movement of the leg portion, facilitating movement and flexibility of the leg of the user. This may allow for a normal human range of movement in the leg of the user, advantageously allowing the leg to be bent whilst seated and standing, and during walking.

The upper leg brace may be configured to provide a clam shell for enclosing at least a portion of a user's thigh. It may be advantageous to enclose the thigh of the user to contain the flesh of the user's thigh. Following a femoral resection or amputation, for example as part of an internal hemipelvectomy, the thigh will naturally compress under load due to the absence of bone. However, by containment of the thigh, it creates a form of support as the flesh has nowhere to go and cannot compress further. The clam shell for containing the thigh may be made of carbon fibre.

Providing a hinge portion in the upper leg brace may be advantageous to facilitate easy donning of the brace by a user. The hinge portion may comprise a flexible material capable of shear loading, configured to permit bending of the hinge portion. This may be advantageous to allow the upper leg brace to hinge for easy donning whilst maintaining shear loading. In some examples, the hinge portion may comprise Kevlar, such as Kevlar with flexible epoxy. The hinge portion may be arranged parallel to the longitudinal axis of the upper leg brace, for example wherein the hinge portion is arranged along the outer side of the leg brace.

The upper leg brace may comprise at least one ratchet strap operable to fasten the upper leg brace around the user's anatomy. This may be advantageous to adjust and secure the exoskeleton to fit the anatomy of the user, optionally enclosing the thigh of the user within the brace for containment. Preferably, the at least one ratchet strap may comprise a magnetic attachment means. This may be advantageous for ease of attachment.

The lower leg brace may be configured to provide a clam shell for enclosing at least a portion of a user's calf, similarly to the clam shell of the upper leg brace described above. The lower leg clam shell may comprise a hinge portion comprising a flexible material capable of shear loading, such as Kevlar with flexible epoxy. This is important to ensure that the ankle joint and engagement with a shoe, discussed in more detail below, remains engaged even under load during ambulating.

The hinge portion may be arranged parallel to the longitudinal axis of the lower leg brace, for example wherein the hinge portion is arranged along the outer side of the lower leg brace. This may be advantageous to improve conformity of the lower leg brace to the user's shin.

The lower leg brace may also comprise at least one ratchet strap operable to fasten the lower leg brace around the user's anatomy. Preferably, the at least one ratchet strap may comprise a magnetic attachment means. This may be advantageous for ease of attachment.

The leg portion may be configured to detachably engage with a shoe worn by the user. This may be advantageous to transfer load captured by the exoskeleton into the shoe. In some examples, the leg portion is configured to detachably engage with the heel portion of a shoe worn by the user.

Existing ankle braces and splints commonly use a rigid plate which is arranged underneath at least a portion of the foot in use and placed into a shoe or used as a part of a "boot". Detachably engaging the exoskeleton with the shoe may be advantageous compared to plate-based solutions as the user maintains flexibility in their foot in the shoe during use. This may improve balance, control of ambulation, and mobility as the user is able to flex their foot in the shoe and accommodate to uneven ground by proprioceptive input, unlike when the foot is interfaced with a rigid plate.

In some examples, the leg portion is configured to detachably engage with a shoe worn by the user via magnets. This may be advantageous for ease of engaging and disengaging the shoe to/fromthe exoskeleton in use.

The leg portion may further comprise an ankle joint. The ankle joint may be configured to be above a portion of the heel of the shoe proximate to the vamp of the shoe. For example, in use, the ankle joint may be positioned either side of the user's ankle. This may be advantageous to allow movement and flexibility of the ankle of the user in use. This may allow for a normal human range of movement in the ankle of the user, advantageously allowing the ankle to be bent whilst seated and standing, and during walking.

The ankle joint may be configured to permit the shoe engaged with the leg portion to rotate relative to the lower calf brace, for example akin to ski bindings. This may be advantageous for ease of engaging and disengaging the shoe to/from the exoskeleton in use.

The ankle joint may further comprise a suspension shock absorbing joint. This may be advantageous to reduce the impact of loading, for example whilst in use, such as during walking.

The suspension shock absorbing joint may be used in conjunction with the sole of the shoe to provide the 'spring'. Additionally, or instead, the suspension shock absorbing joint may be used in conjunction with a carbon fiber shoe insole inserted into the shoe to provide the 'spring'. A carbon fiber shoe insole may be advantageous to reduce or prevent buckling of the shoe under loading. The carbon fiber shoe insole is preferably flexible to allow the user to maintain flexibility in the sole of foot in the shoe during use. This may improve balance, control of ambulation, and mobility as the user is able to flex their foot and the insole and accommodate to uneven ground by proprioceptive input, unlike when the foot is interfaced with a rigid plate.

The torso portion may be configured to enclose at least a portion of the user's torso, for example partially encompassing at least one of the upper, mid, or lower torso. In some examples, the torso portion may comprise a back brace, at least partially enclosing the mid torso, and optionally at least a portion of the upper torso. In other examples, the torso portion may comprise a belt configured to at least partially enclose the mid or lower torso.

In some examples, the torso portion may comprise a back brace configured to provide a clam shell for enclosing at least a portion of a user's torso. Providing a hinge portion in the back brace may be advantageous to facilitate easy donning of the back brace by a user. The hinge portion may comprise a flexible material capable of shear loading, configured to permit bending of the hinge portion. This may be advantageous to allow the back brace to hinge for easy donning whilst maintaining shear loading. In some examples, the hinge portion may comprise Kevlar, such as Kevlar with flexible epoxy.

In some examples, the torso portion comprises at least one ratchet strap. This may be advantageous to adjust and secure the torso portion to fit the anatomy of the user. Preferably, the at least one ratchet strap may comprise a magnetic attachment means. This may be advantageous for ease of attachment.

The torso portion may further comprise at least one hip shield. The at least one hip shield may comprise a rigid portion configured to be arranged in a parasagittal plane of the human body. In use, the at least one hip shield is configured to be arranged at least partially at the height of the hip of the user. Preferably, the at least one hip shield is arranged on the opposite side of the exoskeleton to the exoskeleton hip joint. The hip shield may be advantageous to reduce Trendelenburg gait during ambulation. A Trendelenburg gait is an abnormal gait which often results from a defective hip abductor mechanism, or lack of musculature control, such as gluteal musculature, including the gluteus medius and gluteus minimus muscles. This causes drooping of the pelvis to the contralateral side while walking. This is common following a pelvic resection or internal hemipelvectomy due to the removal of muscle fixing points. This is also common following a high level transfemoral amputation where the muscles and levers are compromised due to the short length of the residual limb.

In some examples, each of the torso arm and the leg arm of the hip joint are configured to engage with a respective dismountable attachment point to fasten the torso arm with the torso portion and the leg arm with the leg portion respectively. In some examples, the dismountable attachment point comprises a mating socket. Each mating socket may comprise a detachable locking fork, wherein the detachable locking fork may be configured to engage with a corresponding mating portion of each of the torso arm and the leg arm of the hip joint. The detachable locking fork may be advantageous for ease of mating of the hip joint to the torso portion and leg portion, facilitating easy donning of the exoskeleton by a user.

The detachable locking fork may comprise a lever arm cam lock mechanism configured to engage with the corresponding mating portion of each of the torso arm and the leg arm of the hip joint. This may be advantageous as the rotational action of the cam lock mechanism may facilitate ease of donning of the exoskeleton by a user, for example by guiding and securing the torso arm of the hip joint to engage with a dismountable attachment point of the torso portion, and the leg arm of the hip joint to engage with a dismountable attachment point of the leg portion.

The detachable locking forks may be configured to operate in a locked configuration and a released configuration, and wherein the detachable locking forks are biased to remain in a locked configuration via magnets.

In another aspect of the disclosure, there is provided an exoskeleton for providing support to a human user having an amputation of at least a portion of the pelvis or legs. The exoskeleton comprises a torso portion and leg portion, the torso portion and leg portion coupled via a hip joint. The leg portion is configured to detachably engage with a shoe worn by the user.

This may be advantageous to transfer load captured by the exoskeleton into the shoe. Existing ankle braces and splints commonly use a rigid plate which is arranged underneath at least a portion of the foot in use and placed into a shoe or used as a part of a "boot". In such configurations, the leg portion does not detachably engage with the shoe itself. Detachably engaging the exoskeleton with the shoe may be advantageous compared to these plate-based solutions as the user maintains flexibility in their foot in the shoe during use. This may improve balance, control of ambulation, and mobility as the user is able to flex their foot in the shoe and accommodate to uneven ground by proprioceptive input, unlike when the foot is interfaced with a rigid plate.

Detachably engaging with the shoe may also be advantageous to utilize the shock-absorption properties of the sole of the shoe. This may be advantageous to reduce the impact of loading, for example whilst in use, such as during walking.

The torso portion may be configured to enclose at least a portion of the user's torso, for example partially encompassing at least one of the upper, mid, or lower torso. In some examples, the torso portion may comprise a back brace, at least partially enclosing the mid torso, and optionally at least a portion of the upper torso. In other examples, the torso portion may comprise a belt configured to at least partially enclose the mid and/or lower torso.

The leg portion may be configured to detachably engage with a portion of the heel of the shoe worn by the user, for example but not limited to via magnets. Detachably engaging with a shoe worn by the user via magnets may be advantageous for ease of engaging and disengaging the shoe to/from the exoskeleton in use.

The leg portion may be further configured to detach from the shoe via rotation of the shoe in the plane of the sole of the shoe. This may be advantageous for ease of engaging and disengaging the shoe to/from the exoskeleton in use, for example akin to ski bindings.

In some examples, the exoskeleton may further comprise a heel receiving portion, wherein the heel receiving portion is configured to be inserted into the heel of a user's shoe. The leg portion is then configured to detachably engage with the heel receiving portion. Preferably, the heel receiving portion is at least partially embedded into the heel of a user's shoe.

In another aspect of the disclosure, there is provided a locking mechanism for fastening a prosthetic or exoskeleton to a user's shoe. The locking mechanism comprises an engagement portion configured to be coupled to the prosthetic or exoskeleton to at least partially support the weight of a user. The locking mechanism also comprises a magnetic receiving portion, wherein the magnetic receiving portion is configured to be inserted into the heel of a user's shoe. The magnetic receiving portion comprises a hemispherical receiving slot in the plane of the sole of the shoe, and wherein the engagement portion comprises a corresponding hemispherical protrusion configured to be inserted into the hemispherical receiving slot to engage the male portion with the magnetic receiving portion. This may be advantageous to detachably engage and secure the prosthetic or exoskeleton to a shoe of the user.

The engagement portion may be configured to detach from the magnetic receiving portion via rotation of at least one of the engagement portion and the magnetic receiving portion in the plane of the hemispherical receiving slot. This may be advantageous for ease of engaging and disengaging the shoe to/from the exoskeleton or prosthesis in use, for example akin to ski bindings.

In another aspect of the disclosure, there is provided a joint for coupling portions of an exoskeleton. The joint comprises a first arm coupled to a centre boss via a first rotation limiting joint having a first range of motion, and a second arm coupled to the centre boss via a second rotation limiting joint having a second range of motion. The second range of motion is greater than the first range of motion.

The joint may further comprise a third arm coupled to the centre boss via a third rotation limiting joint having a third range of motion. Alternatively, or in addition, the joint may comprise an arm, for example a third or fourth arm, directly coupled to the centre boss, wherein the centre boss is configured to rotate.

In some examples, the first, second and third arms are each configured to rotate about the centre boss via a common axis of rotation.

In another aspect of the disclosure, there is provided a joint for coupling portions of an exoskeleton, wherein the joint comprises a first arm coupled to a centre boss via a first rotation limiting joint having a first range of motion, and a second arm coupled to the centre boss via a second rotation limiting joint having a second range of motion. The joint further comprises a corresponding first arm lever arm cam lock attachment point, and a corresponding second arm lever arm cam lock attachment point. Each of the first arm and the second arm comprise a respective detachable locking fork configured to engage with a corresponding mating portion of the corresponding lever arm cam lock attachment point to fasten the respective arm with the respective lever arm cam lock attachment point.

The detachable locking forks may be configured to operate in a locked configuration and a released configuration, and wherein the detachable locking forks are biased to remain in a locked configuration via magnets.

### Drawings

Embodiments of the disclosure will now be described, by way of example only, with reference to the accompanying drawings, in which:
**Fig. 1A** shows a front view of an example exoskeleton on a human user.
**Fig.** 1B shows a side view of the example exoskeleton of Fig. 1A on a human user.
**Fig.** 1C shows a back view of the example exoskeleton of Figs. 1A-1B on a human user.
**Fig. 2A** shows an example hip joint, for example for use within the exoskeleton of Figs. 1A-1C.
**Fig. 2B** shows an exploded view of the hip joint of Fig. 2A.
**Fig. 2C** shows the hip joint of Figs. 2A-2B coupled to sockets, for example for use within the exoskeleton of Figs. 1A-1C.
**Figs. 3A-3D** illustrate the range of motion of the hip joint, for example the hip joint of Figs. 2A-2C.
**Fig.** 4 shows an example socket, for example the socket of Fig. 2B for connection of a hip joint to an exoskeleton.
**Figs. 5A-5H** illustrate the coupling of an arm of a hip joint, such as the hip joint of Fig. 2A, to a socket, such as the socket of Fig. 4. **Fig. 5I** shows a cross-section of the coupling of Figs. 5A-5H, between an arm of the hip joint and the socket.
**Fig. 6** shows an example ankle joint, for use in an exoskeleton, such as the exoskeleton of Figs. 1A-1C, including a magnetic shoe interface.
**Fig. 7A** shows the magnetic shoe interface of Fig. 6 installed in a shoe.
**Fig. 7B** shows the magnetic shoe interface of Fig. 7A coupled to the ankle joint of Fig. 6.
**Figs. 8A and 8B** show an example rotation restricting part, for example for use within the hip joint of Figs. 2A, 2C, and 3A-D.
**Fig. 8C** shows the rotation restricting part of Figs. 8A and 8B, with a top cap of the hip joint.
**Figs. 8D and 8E** show cross-sections illustrating the range of motion of a hip joint comprising the rotation restricting part.

### Specific description

Embodiments of the claims relate to an exoskeleton for providing support to a human user. It will be appreciated from the discussion above that the embodiments shown in the Figures are merely exemplary, and include features which may be generalised, removed or replaced as described herein and as set out in the claims.

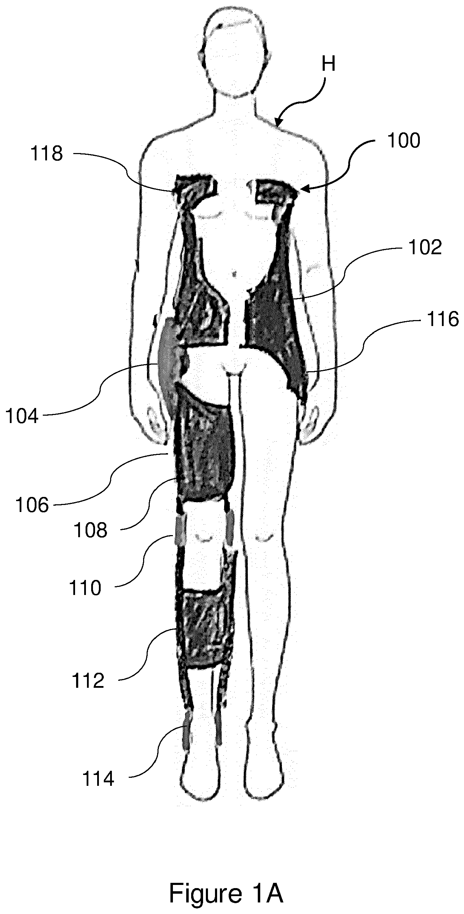

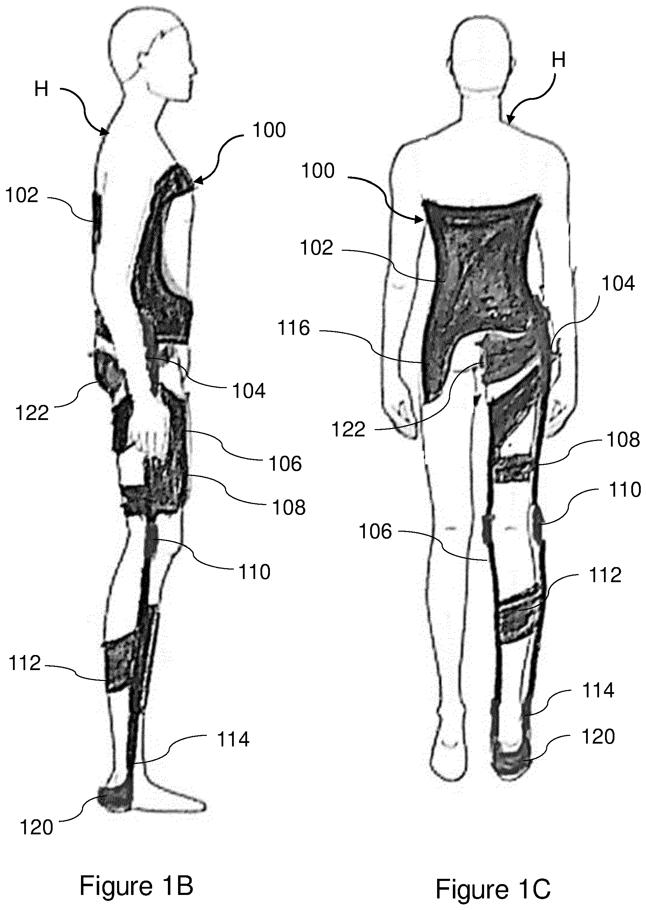

**Figs. 1A-1C** show an example exoskeleton 100 as worn by a human user H. The exoskeleton 100 comprises a torso portion 102 and a leg portion 106, coupled via a hip joint 104.

The torso portion 102 comprises a back brace that at least partially encloses the upper and mid-section of the torso. In this example, the back brace comprises a substantially carbon fiber structure. The torso portion 102 further comprises a pair of underarm supports 118, arranged on opposite lateral sides of the torso portion. Each underarm support 118 comprises a deformable moulding, for example a silicon moulding. The deformable mouldings may be advantages to protect the axillary nerve of the human user H in use.

The torso portion 102 may additionally comprise at least one ratchet strap (not shown). Preferably, each ratchet strap comprises a magnetic attachment means. This may be advantageous to secure the torso portion to the torso of the human user H and prevent the torso portion H collapsing forward.

The torso portion 102 further comprises a hip shield 116. The hip shield 116 comprises a rigid part arranged on the opposite side (or contralateral side) of the exoskeleton 100 to the hip joint 104. The hip shield 116 is partially arranged in a parasagittal plane of the human body, at the height of the hip of the human user H. In this example, the hip shield 116 is integral to the torso portion 102, however in other examples the hip shield 116 may be detachable.

The torso portion 102 also comprises a hinge (not shown) on the posterior surface of the torso portion 102, adjacent to the back of the human user H. The hinge is arranged parallel to the craniocaudal axis of the human user H, preferably in the sagittal plane. In this example, the hinge portion comprises Kevlar and flexible epoxy to permit bending of the hinge portion whilst maintaining shear loading between the carbon fiber segments of the torso portion 102 on either side of the hinge.

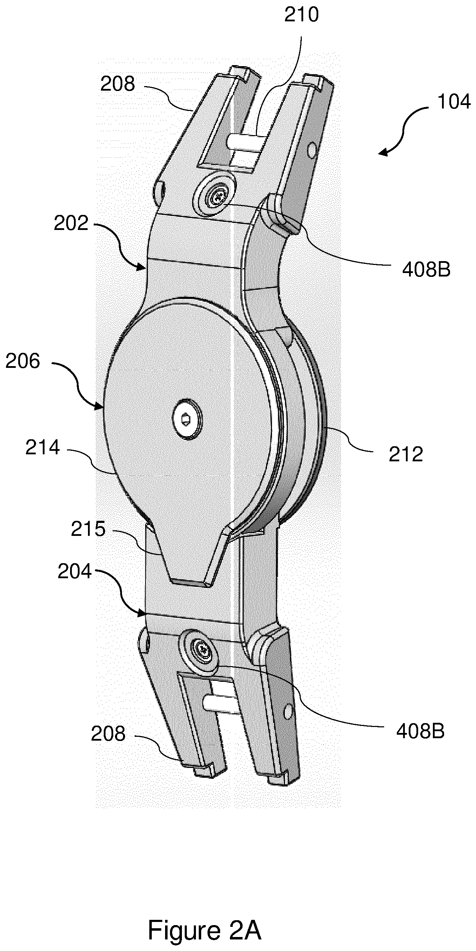

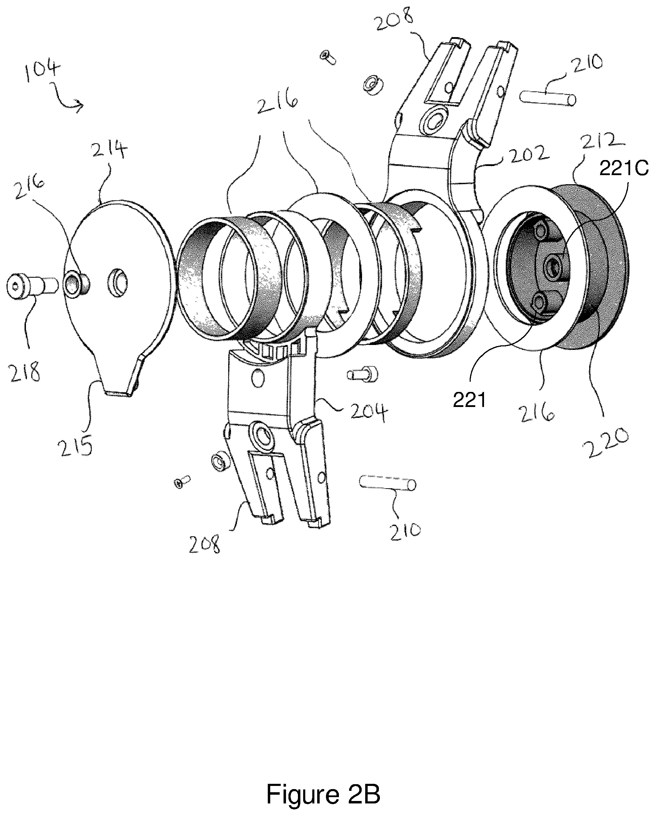

The hip joint 104 is arranged on the ipsilateral side of the exoskeleton as the leg portion 106. An example hip joint 104 is shown in more detail in Figs. 2A and 2B.

The leg portion 106 further comprises an upper leg brace 108 and a lower leg brace 112. The upper leg brace 108 and the lower leg brace 112 are coupled together by a knee joint 110. The upper leg brace 108 and the lower leg brace 112 each comprise at least one ratchet strap (not shown). Preferably, each ratchet strap comprises a magnetic attachment means.

The upper leg brace 108 and lower leg brace 112 each comprise a pair of carbon fiber supports. Each carbon fiber support of the pair is arranged in a parasagittal plane, on opposite sides of the leg. Both the upper leg brace 108 and lower leg brace 112 further comprise carbon fiber cross bracing between the pair of carbon fiber supports.

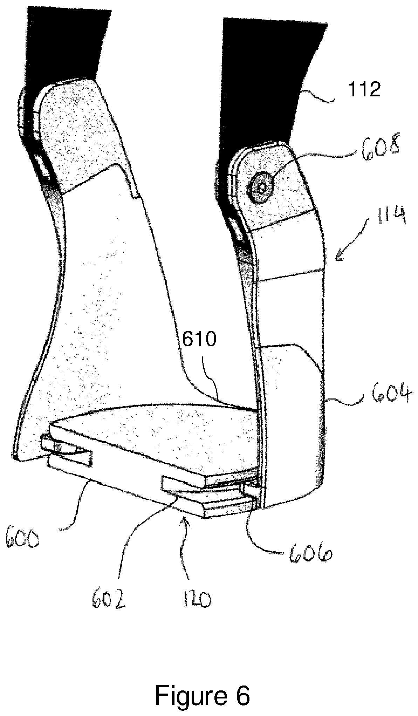

An ankle joint 114 is coupled to the distal end of the lower leg brace 112. An example ankle joint 114 is shown in more detail in Fig. 6.

As shown in Figs. 1B-1C, the exoskeleton 100 further comprises a buttock support portion 122. The buttock support portion 122 comprises a curved plate configured to encompasses the lower and mid buttock of the user H from the midpoint of the back at the gluteal sulcus to the upper front of the thigh at the top of the rectus femoris muscle with the lower edge following the line of the gluteal sulcus. In this example, the buttock support portion is made from carbon fiber.

The torso portion 102 is configured to enclose at least a portion of a human user H's torso and capture the vertical load of the human user H's upper body. In particular, the pair of underarm supports 118 are configured to capture the vertical load of the user H's upper body. The hinge portion of the torso portion 102 is configured to provide a clam shell for easy donning of the exoskeleton 100.

The buttock support portion 122 is also configured to capture vertical load, in particular vertical load from the mid body. This may reduce, and in some examples eliminate, load capture at the base of the axilla of the human user H (under the armpits) which can be uncomfortable and damaging to the axillary nerve of the user.

Both the torso portion 102 and buttock support portion 122 are configured to transfer the captured vertical load to the hip joint 104.

The upper leg brace 108 is configured to at least partially encompass the thigh of human user H. The upper leg brace 108 is also configured to transfer the load from the hip joint 104 to the knee joint 110.

The knee joint 110 is configured to rotate such that the upper leg brace 108 is moveable relative to the lower leg brace 112. The knee joint 110 is also configured to transfer the load from the upper leg brace 108 to the lower leg brace 112.

The lower leg brace 112 is configured to at least partially encompass the calf of human user H. The lower leg brace 112 is also configured to transfer the load from the knee joint 110 to the ankle joint 114.

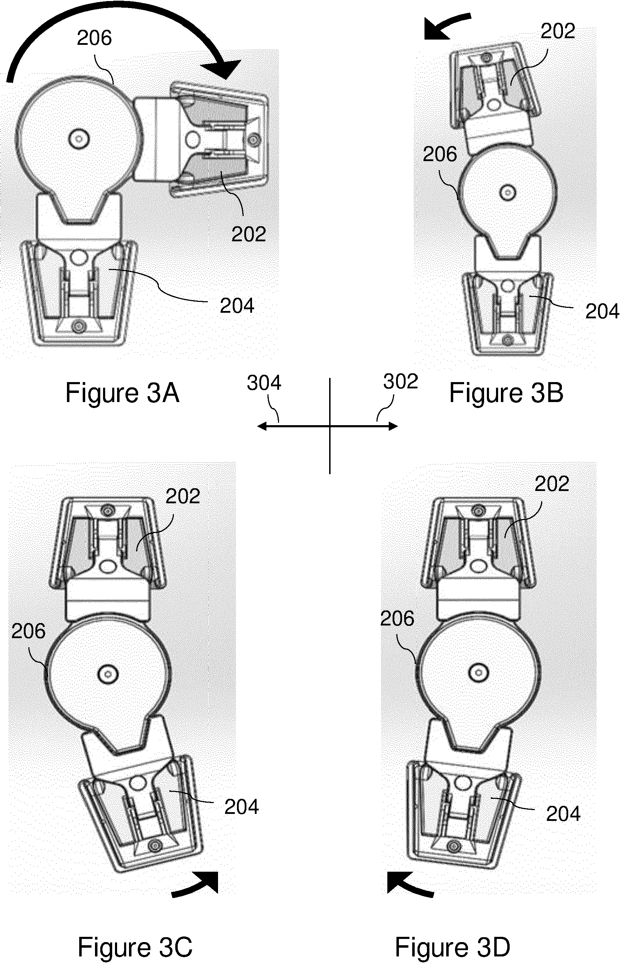

The torso portion 102, the leg portion 106 and the buttock support portion 122 are configured to rotate about a common axis of the hip joint 104. The degrees of rotation are constrained independently of each other allowing specific degrees of rotation of (i) the torso portion 102 relative to the hip joint 104, (ii) the buttock support portion 122 to the hip joint 104, and (iii) the upper leg portion 108 to the hip joint 104. This is shown in more detail in Figs. 3A to 3D.

The rachet straps are configured to adjust and fasten the respective portion around the human user's anatomy.

The hip shield 116 is configured to encompass the hip and a portion of the upper thigh on one side of the human user H's body. The hip shield 116 is configured to inhibit outward lateral movement of the hip relative to the torso portion 102. This may be by advantageous to reduce hip swing during ambulation.

In use, the torso portion 102 and buttock support portion 122 capture the vertical load of the user H's upper and mid body and transfer the load to the hip joint 104.

The hip joint 104 transfers the total load to the leg portion 106, from the upper leg brace 108, via the knee joint 110, to the lower leg brace 112.

The example shown in Figs. 1A-1C comprises a single hip joint 104 and a single leg portion 106, wherein the hip joint 104 is arranged on the ipsilateral side of the exoskeleton 100 as the leg portion 106. However, the skilled person will understand, that other example exoskeletons may provide two leg portions 106 and two hip joints 104, wherein each leg portion is coupled to a respective hip joint. Such a configuration may be particularly advantageous for use by individuals with double lower limb amputations, or patients who are unable to have a bilateral hip replacement, for example due to age. In examples comprising two hip joints 104, the second hip joint may replace the hip shield 116, or each hip joint may instead be coupled to or integrated within a hip shield, for example wherein the hip shield is in the same parasag ittal plane as the respective hip joint.

An example hip joint 104 is shown in more detail in **Fig. 2A****.** The hip joint 104 comprises a torso arm 202 and a leg arm 204. Both the torso arm 202 and leg arm 204 are coupled at a proximal end to a central rotating portion 206 of the hip joint 104 via respective rotation limiting joints. The central rotating portion 206 comprises an end cap 212 and a top cap 214, wherein the top cap is arranged opposite to the end cap 212.

Each of the torso arm 202 and the leg arm 204 of the hip joint 104 comprise a mating portion. The mating portion 208 is arranged at the distal end of each of the torso arm 202 and the leg arm 204. In this example, the mating portion 208 comprises an attachment bar 210 coupled in between a pair of parallel members. The attachment bar 210 is perpendicular to the pair of parallel members, wherein the pair of parallel members are parallel to the longitudinal axis of the respective torso arm 202 or leg arm 204. The mating portion 208 has a slight curvature, such that the outermost end of the mating portion curves towards the end cap 212.

The hip joint 104 is preferably made of metal, such as aluminium. However, the skilled person will understand that other materials may be used. In some examples, the hip joint is manufactured via 3D printing. Optionally, the metal is anodised, such as anodised aluminium. However, the skilled person will understand that other methods of manufacture can be used.

**Fig. 2B** shows an exploded view of the hip joint 104 of Fig. 2A. The end cap 212 comprises a substantially circular shape with a central boss 220. The end cap 212 also comprises a plurality of subsidiary bosses 221, arranged within the interior of the central boss 220. One of the subsidiary bosses 221C is arranged centrally to the end cap 212, having a smaller diameter than the central boss 220. A portion of the subsidiary bosses 221 are coupled to the internal circumference of the central boss 220. Bearings 216 are arranged between the each of the torso arm 202, leg arm 204, and the central boss 220 of the end cap 212.

The top cap 214 comprises a substantially circular shape with a radial protrusion 215. The top cap 214 is coupled to the leg arm 204 via the radial protrusion 215.

A shoulder screw 218 and bearing 216 couples the top cap 214 to the end cap 212, thereby also securing each of the arms 202 and 204 within the hip joint 104. The shoulder screw 218 is configured to be received by the centrally positioned subsidiary boss 221C.

The torso arm 202 is configured to detachably couple to a torso portion, such as torso portion 102 shown in Figs. 1A-C. Similarly, the leg arm 204 is configured to detachably couple to a leg portion, such as leg portion 106 shown in Figs. 1A-C. The end cap 212 is configured to detachably couple to a buttock support portion, such as buttock support portion 122 shown in Figs. 1B-C.

The rotation limiting joint for the torso arm 202 is configured to have a first range of motion about the central boss 220, as shown in **Figs. 3A to 3B****.** The rotation limiting joint for the leg arm 204 is configured to have a second range of motion about the central boss 220, as shown in **Figs. 3C to 3D****.** The top cap 214 is configured to rotate relative to the end cap 212, and the coupling between the top cap 214 and the leg arm 204 means that the top cap 214 rotates in accordance with the rotation of the leg arm 204.

The second range of motion is less than the first range of motion. The second range of motion about the central boss 216 does not overlap with the first range of motion. The rotational limiting joints are configured to allow for a normal human range of movement, advantageously allowing the leg portion 106 to move relative to the torso portion 102 whilst seated, standing, and during walking. The rotation limiting joints may also be advantageous to prevent over-rotation / hyperextension of the leg portion 106 relative to the torso portion 102, and vice versa. This may provide support to the human user H in use and 30 prevent collapse of the exoskeleton 100.

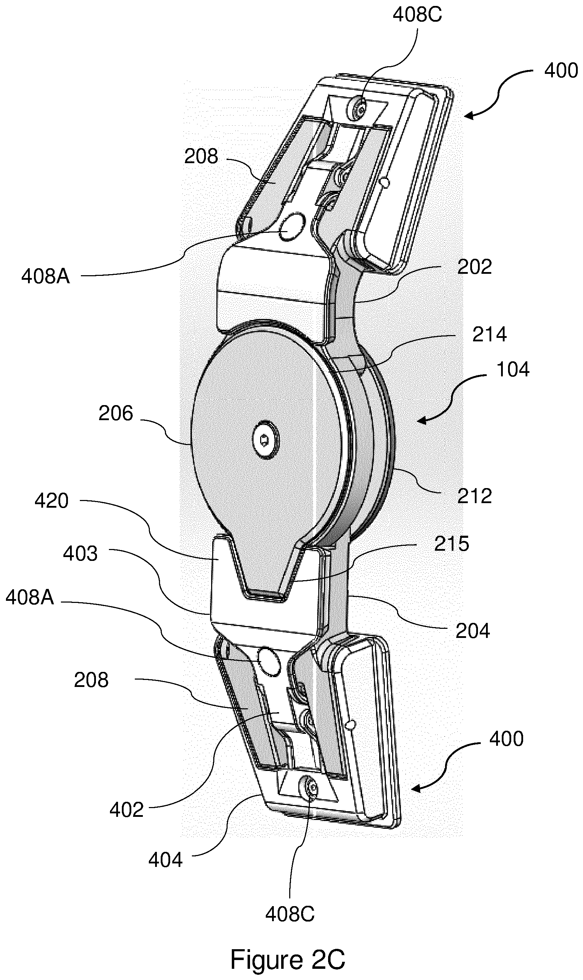

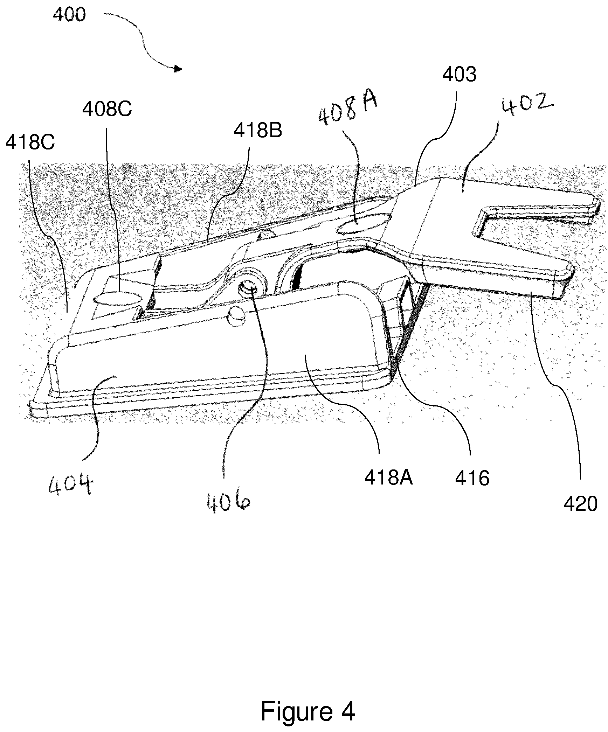

As shown in **Fig. 2C****,** each mating portion 208 is configured to engage with a corresponding mating socket 400. An example mating socket 400 is shown in more detail in Fig. 4. Mating sockets 400 are coupled to the torso portion 102 and leg portion 104 respectively, such that the mating socket 400 is configured to fasten the torso arm 202 with the torso portion 102 and the leg arm 204 with the leg portion 106 respectively. The mating portion 208 and detachable mating socket 400 may be advantageous for ease of mating of the hip joint 104 to the torso portion 102 and leg portion 106, facilitating easy donning of the exoskeleton 100 by a human user H.

The hip joint 104 is configured to operate in a parasagittal plane of the human body, such that the rotation of the torso arm 202 and the leg arm 204 is in a parasagittal plane.

The buttock support 122 has 30 degrees of total movement relative to the leg arm 204. The leg arm 204 can move 20 degrees in a first direction and 10 degrees rearwards in a second direction before the central rotating portion 206 coupled to the buttock support 122 is forced to move with the leg arm 204.

**Figs. 3A to 3B** show the first range of motion of the rotation limiting joint for the torso arm 202 about the central rotating portion 206. Assuming the central rotating portion 206 remains still, the torso arm 202 is configured to rotate up to approximately 90 degrees in a first direction 302 and less than 10 degrees in a second direction 304, wherein the second direction 304 is opposite to the first direction 302. In a preferred embodiment, the torso arm 202 is configured to rotate 0 degrees in the second direction 304.

**Figs. 3C to 3D** show the second range of motion of the rotation limiting joint for the leg arm 204 about the central rotating portion 206. Assuming the central rotating portion 206 remains still, the leg arm 204 is configured to rotate approximately 20 degrees in a first direction relative to the torso arm 202 and approximately 10degrees in a second direction, wherein the second direction is opposite to the first direction.

In use, the first direction 302 is parallel to the anterior direction, and the second direction 304 is parallel to the posterior direction. The range of rotation of each of the torso arm 202 and leg arm 204 is measured from a plane parallel to the frontal plane, in the respective anterior or posterior direction.

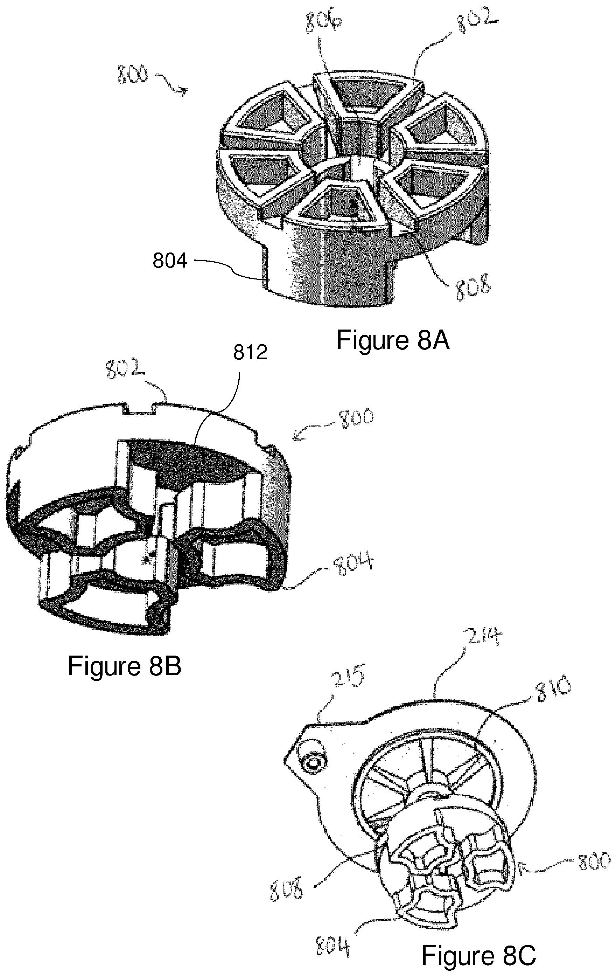

Alternatively to the exploded view of the hip joint 104 shown in Fig. 2B comprising the bearings 216, the restriction of the hip joint 104 may instead be provided by a rotation restriction part 800, as shown in **Figs. 8A-8B****.** The part 800 has a generally circular profile, comprising a central aperture 806. Fig. 8A shows a first surface of the rotation restriction part 800. The first surface comprises a plurality of raised segments 802, arranged around the circumference of the part 800. Neighbouring segments 802 are each separated by a channel 808.

As shown in **Fig. 8C****,** the first surface of the rotation restriction part 800 is configured to be arranged adjacent to the top cap 214 of the hip joint 104. Each channel 808 is configured to receive a rib 810 on the internal surface of the top cap 214. The raised segments 802 constrain the ribs 810 of the top cap 214 within the corresponding channel 808 of the rotation restriction part 800, thus coupling the rotation restriction part 800 to the top cap 214 such that rotation of the top cap 214 drives rotation of the rotation restriction part 800 and vice versa. The number of raised segments 802 and channels 808 on the first surface of the part is dependent on the number of ribs 810 of the top cap 214.

**Fig. 8B** shows a second surface of the rotation restriction part 800, opposite to the first surface. The second surface comprises a second plurality of raised segments 804 arranged around the circumference of the part 800. In the example shown in Fig. 8B, the second plurality of raised segments comprises three raised segments 804. The separation 812 between neighbouring segments of the second plurality of raised segments 804 is greater than the separation between neighbouring segments of the first plurality of raised segments 802. Each side wall of the second plurality of raised segments 804 comprises a concave portion, wherein the side wall is defined as the wall of each segment which extends in a substantially radial direction relative to the circular profile of the part 800.

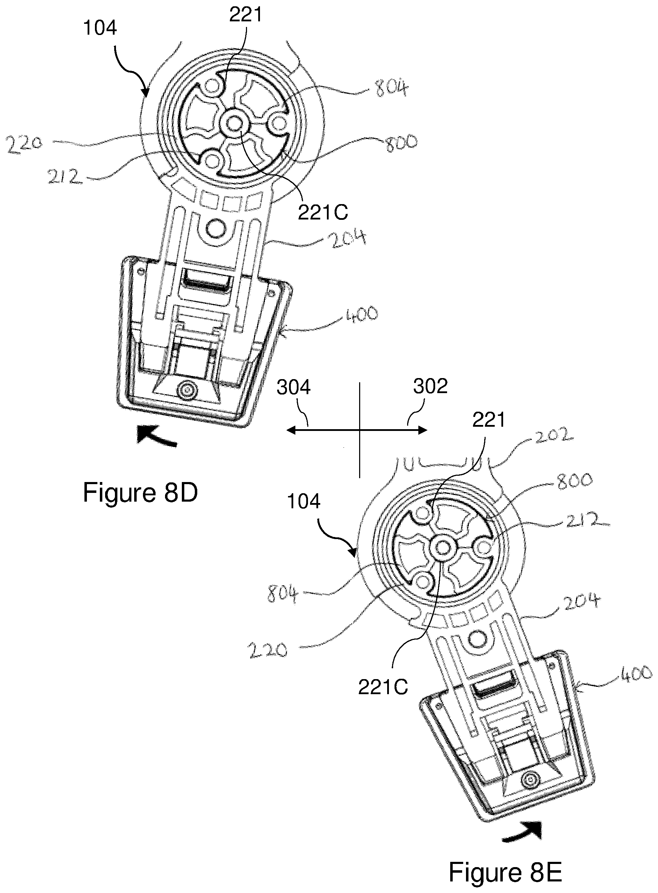

**Figs. 8D and 8E** show the rotation restriction part 800 in use in a hip joint 104. Similarly, as shown in **Figs. 3C to 3D****,** the leg arm 204 is configured to have a range of motion about the central boss 220. The coupling between the top cap 214 and the leg arm 204, as well as the coupling between the top cap 214 and the rotation restriction part 800 means that the rotation restriction part 800 rotates in accordance with the rotation of the leg arm 204.

The top cap 214 is also configured to rotate relative to the end cap 212, wherein the end cap 212 is coupled to the buttock support 122, The buttock support 122 has 30 degrees of total movement relative to the leg arm 204.

As shown in **Figs. 8D and 8E****,** the rotation restriction part 800 restricts independent movement of the leg arm 204 as the concave portions of the raised segments abut against the subsidiary bosses 221 on the end cap 212. The leg can move 20 degrees in an anterior direction 302, and 10 degrees in a posterior direction 304 before the central boss 220, and therefore buttock support 122, is forced to move with the leg arm 204.

The rotation restriction part 800 may be 3D printed. This may be advantageous to easily and cheaply change the angles of restriction relative to the leg arm 204 by simply printing a new rotation restriction part 800 as required with different separations 812 between the neighbouring segments 804. This provides the flexibility to reduce the movement down to zero degrees relative to the leg arm 204 if required, for example wherein there is no separation 812 between neighbouring segments 804, or, at the other extreme if the part is removed completely, the buttock support 122 would rotate with complete independence from the leg arm 204.

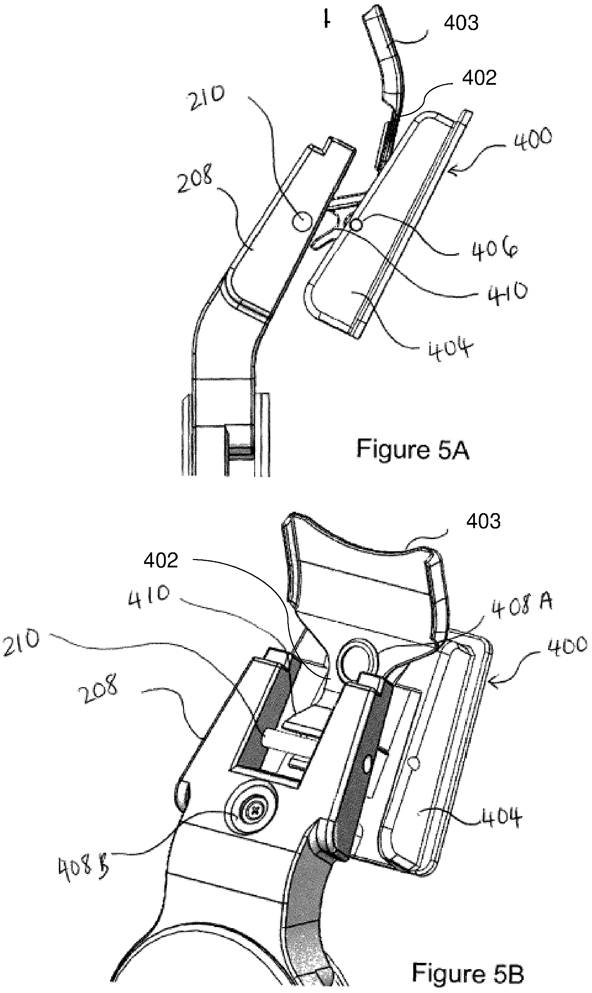

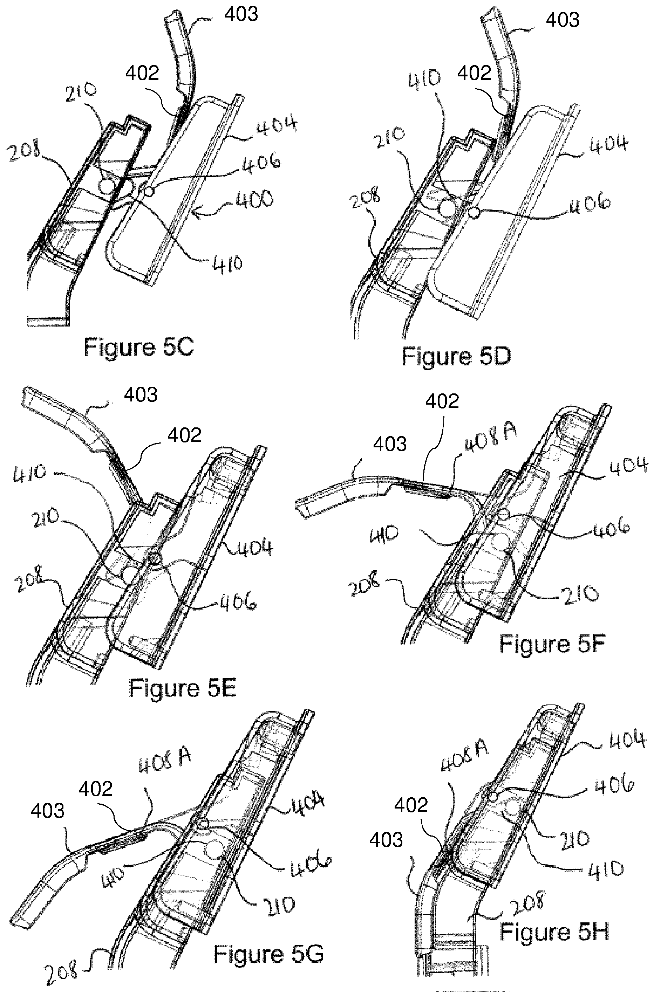

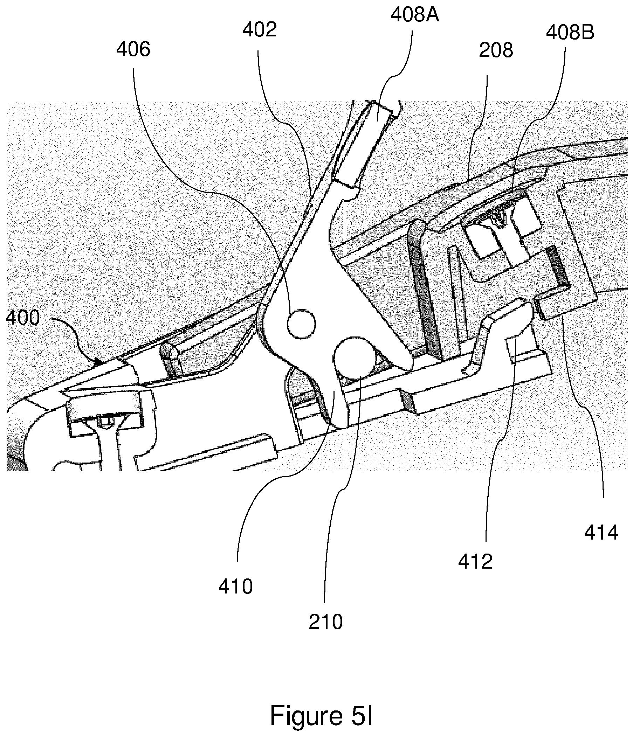

**Fig. 4** shows an example mating socket 400. The mating socket 400 comprises socket housing 404, and a lever arm cam lock mechanism. In this example, the socket housing 404 has a substantially trapezoid shape, comprising a base 416 and three side walls 418A-C. The side walls 418A-C comprise a pair of opposing sloping side walls 148A, 418B, and an end wall 418C, wherein the end wall 418C is arranged along the short parallel edge of the trapezoid. The lever arm cam lock mechanism comprises a lever arm 402, wherein the lever arm 402 comprises an actuator portion 403 at the distal end and a locking fork 410 (as shown in more detail in Fig. 5I) at the proximal end. The lever arm 402 further comprises a pivot 406, disposed between the actuator portion 403 and the locking fork 410. The pivot point 406 is also coupled to the socket housing 404. The lever arm 402 is shaped such that the locking fork 410 extends in a direction substantially orthogonal to the actuator portion 403. The actuator portion 403 is slightly curved in a direction towards the locking fork 410.

The locking fork 410 (as shown in more detail in Fig. 5I) comprises two prongs, defining a receiving portion therebetween.

In the example shown in Figure 4, the socket 400 is configured for attachment to the leg arm 204 of the hip joint 104. The distal end of the actuator portion 403 comprises a pair of prongs 420, wherein the pair of prongs 420 are configured have a complementary shape to the radial protrusion 215 of top cap 214. In the locked configuration, the prongs 420 are configured to surround the radial protrusion 215, as shown in Fig. 2C. Alternatively, a socket 400 configured for attachment with the torso arm 202 may comprise a concave shape at the distal end of the actuator portion 403. The concave shape is configured to have a complementary shape to the central portion 206, as shown in Fig. 2C.

The pivot 406 is configured to rotate such that the lever arm 402 is configured to rotate relative to the socket housing 404.

The locking fork 410 may be configured to operate in a locked configuration and a released configuration, wherein the locking fork 410 is transitioned between the locked configuration and the released configuration by rotation of the pivot 406 actuated via rotation of the actuator portion 403. The socket 400 in Figure 4 is pictured in the locked configuration.

The socket 400 is configured to receive and engage with an arm of a hip joint 104, as shown in Figs. 5A to 5H. The arm of the hip joint is configured to be received along the long parallel edge of the trapezoid socket housing 404. In particular, the locking fork 410 is configured to receive and engage with the attachment bar 210 of an arm of the hip joint 104, such that the attachment bar 210 is received by the receiving portion between the two prongs of the locking fork 410.

The curvature of the actuator portion 403 is substantially the same as the curvature of the mating portion 208 of the arm 204 or 206, such that, in the locked configuration in use, the actuator portion 403 lies flush against the outer surface of the mating portion 208, as shown in Fig. 5H.

The socket housing 404 is also configured to attach to at least one of the torso portion 102, or the leg portion 106. In some examples, the socket housing 404 may be configured to reversibly attach and detach.

As shown in **Figs. 5A to 5D****,** in use, the locking fork 410 receives and engages with the attachment bar 210 in the released configuration. In use, the locking fork 410 is biased to remain in the released configuration via at least one magnet 408A on the lever arm 402, wherein the magnet 408A engages with a second magnet 408C arranged on the socket housing 404, proximate to the end wall 418C. This may be advantageous to hold the lever arm 402 open in the released configuration such that the attachment bar 210 of the mating portion 208 may be easily aligned with the locking fork 410. This may be particularly advantageous for the mating socket 400 configured to receive the torso arm 202, wherein the magnetic engagement between magnets 408A and 408C is configured to hold the lever arm 402 open in the released configuration against gravity whilst the torso arm 202 is being fitted.

The actuator portion 403 of the lever arm 402 is then rotated about the pivot 406 towards the mating portion 208 of the arm of the hip joint 104, as shown in **Figs. 5E to 5G****.** As a result, the locking fork 410 is transitioned into the locked configuration and the mating portion 208 of the arm is drawn into the socket housing 404. The cam lock action of the locking fork 410 results a horizontal and a vertical force component being applied to the attachment bar 210 which draws the mating portion 208 into the socket 400. The socket housing 404 comprises a bottom engagement portion 412 comprising a lip (as shown in Fig. 5I). As the mating portion 208 is drawn into the socket 400, the bottom engagement portion 412 is configured to engage with a corresponding bottom engagement portion 414 on the torso arm 202 or leg arm 204.

As show in **Fig. 5H****,** the mating portion 208 is then secured within the socket 400 as the locking fork 410 is secured in the locked configuration. In use, the locking fork 410 is biased to remain in the locked configuration via at least one magnet 408A, wherein the magnet 408A engages with the mating portion 208 of the respective arm of the hip joint 104. Preferably, the mating portion 208 also comprises a magnet, such as magnet 408B, to increase the strength of the magnetic engagement.

The mating portion 208 is configured to be detached from the socket 400 by rotating the actuator portion 403 of the lever arm 402 about the pivot 406 in the opposite direction, away from the mating portion 208 of the arm of the hip joint 104. As a result, the locking fork 410 is transitioned into the released configuration and the mating portion 208 of the arm is drawn out of the socket housing 404. The cam lock action of the locking fork 410 results an opposite horizontal and a vertical force component being applied to the attachment bar 210 which draws the mating portion 208 out of the socket 400.

**Fig. 6** shows an example ankle joint 114, such as the ankle joint 114 of Figs. 1A to 1C, and magnetic shoe engagement portion 120.

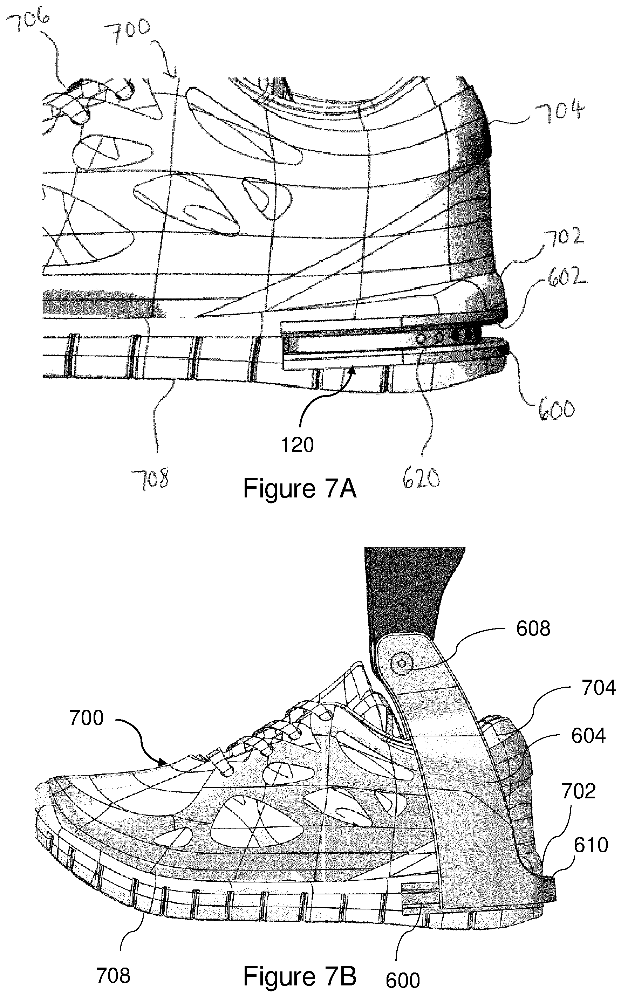

The ankle joint 114 comprises a pair of substantially vertical members 604, positioned either side of the ankle of a human user H. The pair of substantially vertical members 604 are coupled at the distal end by a substantially horizontal member 610. The horizontal member 610 is curved such that it is configured to fit around the back of the heel of a human user H's shoe, for example as shown in more detail in Figure 7B.

The proximal end of each vertical member 604 is coupled to the lower leg brace 112 by a pivot 608. The proximal end of each vertical member 604 is slightly curved in the anterior direction such that the pivot 608 sits forwards of the magnetic shoe engagement portion 120.

The distal end of the ankle joint 114, comprising the horizontal member, is coupled to the magnetic shoe engagement portion 120.

The ankle joint 114 is preferably made of metal, such as aluminium. Optionally, the metal is anodised, such as anodised aluminium. However, the skilled person will understand that other materials may be used. In some examples, the ankle joint 114 is manufactured via 3D printing. However, the skilled person will understand that other methods of manufacture can be used.

The horizontal member at the distal end of the ankle joint 114 comprises a magnetic rail 606. In this example, the rail 606 is metal, comprising a plurality of neodymium magnets, however the skilled person will understand that other types of magnets may be used, or alternatively the rail 606 may be made from a magnetic material.

The magnetic shoe engagement portion 120 comprises a plate 600. The plate 600 has a substantially semi-circular or semi-ellipse shape. A slot 602 is arranged around the curved perimeter of the plate 600. At least a portion of the slot 602 is magnetic, preferably comprising a plurality of magnets 620 as shown in Fig. 7A. The magnets 620 are arranged such that their polarity is in an alternating pattern around the slot 602. In this example, the plate 600 is metal, comprising a plurality of neodymium magnets 620, however the skilled person will understand that other types of magnets may be used. The plate 600 may be manufactured by way of 3D printing, however the skilled person will understand that other methods of manufacture may be used.

The pivot 608 is configured to allow movement of the foot and shoe relative to the lower leg brace 112.

The plate 600 is sized to be at least partially embedded in the sole of a user's shoe, wherein at least a portion of the slot 602 is arranged along an external surface of the shoe. An example configuration is shown in more detail in Fig. 7A.

The rail 606 is configured to fit within the slot 602 of the shoe engagement portion 120. As such, the curved edge of the plate 600 is configured to be concentric with the curved horizontal member 610 of the ankle joint 114. The slot 602 is configured to reversibly and magnetically engage with the rail 606. The rail 606 comprises magnets arranged such that their polarity is in an alternating pattern around the horizontal member 610, wherein the alternating pattern is opposite to that of the magnets 620 of the slot 602.

**Fig. 7A** shows an example shoe 700 comprising a shoe engagement portion 120. The shoe 700 comprises a sole 708 arranged along the bottom surface of the shoe, opposite to the vamp 706 of the shoe. The back portion of the sole 708 comprises the heel 702 of the shoe, wherein the heel 702 is arranged adjacent to the heel counter 704 at the rear of the shoe 700.

The plate 600 is embedded within the heel 702 of the shoe 700 such that the slot 602 is accessible. The slot 602 is arranged around the heel 702 of the shoe, proximate to the heel counter 704.

The plate 600 may be retrofitted into the heel 702 of a shoe 700, or a shoe may be manufactured comprising the plate 600 embedded within the heel 702.

**Fig. 7B** shows the shoe 700 in magnetic engagement with the ankle joint 114. The pair of substantially vertical members 604 are positioned opposite sides the shoe 700, close to the heel counter 704. The horizontal member 610 is arranged around the back of the heel 702 of the shoe 700. The pivot 608 sits forwards of the magnetic shoe engagement portion 120 and heel counter 704 of the shoe 700.

The ankle joint 114 is configured to receive the vertical load from the lower leg brace 112. The shoe engagement portion 120 is configured to take the vertical load from the ankle joint 114 and transfers the vertical load into the sole of the shoe, which ultimately transfers the vertical load to the ground.

The ankle joint 114 is also configured to create a suspension shock absorbing joint, using the sole 708 of the shoe 700 to provide a 'spring'. Optionally, a flexible carbon fiber insole may also be inserted into the shoe 700 (not shown). The carbon fiber insole may also be used in conjunction with the sole 708 to create the suspension shock absorbing joint.

In use, the shoe 700 is configured to detachably engage with the ankle joint 114 by rotating in the plane of the sole 708 of the shoe 700, for example akin to ski bindings. This may be advantageous for ease of disengaging the shoe 700 to/from the ankle joint 114, and exoskeleton 100 in use.

For example, as the shoe 700 is offered up to the ankle joint 114 such that the curved edge of the plate 600 is configured to be concentric with the curved horizontal member 610 of the ankle joint 114, the polarity of the alternating magnets of the slot 602 and rail 606 are all aligned and magnetically attracted to each other, thus holding the shoe to the exoskeleton in the correct position. Upon twisting the shoe 700 in the plane of the sole 708 of the shoe 700, the alignment of the magnets is changed such that they now oppose each other, and the shoe 700 is repelled from the ankle joint 114 and released.

The embodiments described above relate to an exoskeleton, comprising a hip joint, for providing support to a human user. However, the skilled person will understand that the hip joint, such as hip joint 104 shown in Fig. 2A may also be used to provide support to a human user in other arrangements, configurations, or use cases.

For example, the hip joint 104 may be used for lower limb amputees. In one example, a mating socket 400 is coupled to a prosthesis of a user, for example a prosthetic leg. The hip joint 104 may then be reversibly coupled to the prosthetic leg via the mating socket 400. A torso portion, and optionally a buttock support 116, may be reversibly coupled to the hip joint 104 as desired in order to provide additional support to the user when needed. The torso portion may comprise a back brace, at least partially enclosing the mid torso, and optionally at least a portion of the upper torso, such as back brace 102 described above. Alternatively, the torso portion may comprise a belt portion configured to at least partially enclose the mid or lower torso, for example wherein the belt portion is configured to be rigid, for example made from carbon fibre.

This may offer a number of advantages for lower limp amputees, such as prolonging the period that the user can stand without tiring and reducing loading at the interface between the stump and the prosthesis.

The risk of falling may also be reduced. This is a common issue for prosthetic leg wearers with potentially severe implications. One of the key risk factors is that amputees do not always know the exact length of their leg. Despite best attempts to secure a prosthetic leg to the residual limb, for example using liners and suction, a degree of 'pistoning' of the residual limb inside the prosthetic socket remains as the user continually loads and unloads the leg, particularly during the swing phase of the prosthetic leg when walking. Thus, the overall length of the leg can vary, and this variance can result in catching the toe or the heel, resulting in a stumble or potentially resultant fall. This is also often affected by sweat, how vigorous the activity is, or even the amputee's level of hydration which can affect how well the socket fits.

By providing the torso portion coupled via the hip joint to the prosthetic leg, this may reduce the 'pull out' of the residual limb relative to the socket of the prosthetic leg. This in turn may provide a non-varying overall leg length and a better level of control.

The tendency of the residual limb to rotate around the vertical axis inside the prosthetic socket may also be reduced by coupling the prosthesis to the torso portion via the hip joint. This problem is exacerbated by sweat or if the socket of the prosthetic is ill-fitting. In extreme cases, this can even cause the prosthetic leg to fall off. Currently, individuals may resort to use of a soft belt around the waist to try and restrain the leg, however the soft belt cannot control twisting. Coupling the prosthetic to a rigid torso portion via the hip joint, such as a back brace, or rigid carbon fibre belt portion, may help to reduce twisting.

By offsetting some of the individual's weight via the hip joint and improving control over even a poorly fitting socket, this could result in a significant reduction in the number of sockets required by a patient - each of which can cost thousands of pounds and requires multiple trips to the prosthetist. This has clear advantages both for the individual and health authorities.

Furthermore, early stages of learning to use a prosthetic leg can be very difficult and fraught with challenges as the residual limb changes shape significantly over time and there is the ever-present severe risk of falling. The torso brace and hip joint connected to the prosthetic leg may facilitate the learning process considerably by providing better control even with an ill-fitting socket. For example, adduction and abduction may be restricted, and forward and backward degrees of rotation can be controlled via the hip joint. This could significantly reduce the rehabilitation process, enabling more patients to use a prosthetic leg sooner and begin ambulating much earlier with the resultant health benefits from exercise and mobility.

Another example embodiment and use case may comprise a load-bearing ankle brace. The ankle brace may comprise a lower leg brace, such as lower leg brace 112, configured to enclose at least a portion of the user's lower leg. The brace may be made at least partially of carbon fibre, for example comprising rigid carbon fibre portions secured with a ratchet strap that wraps around the lower leg. The lower leg brace may be configured to provide a clam shell for ease of donning, including a flexible hinge portion. An ankle joint, such as the ankle joint 114 as shown in Figure 6, is coupled to either side of the lower leg brace, on opposite sides of the user's ankle. The ankle joint comprises a shoe engagement portion, such as the shoe engagement potion 120 shown in Figure 6. By capturing at least a portion of the vertical force from walking and transferring it to the user's shoe via the shoe engagement portion, bypassing the user's own ankle, such a load-bearing ankle brace could be used in the treatment of weak, injured, or arthritic joints in the ankle or foot. A flexible carbon fibre shoe insole may additionally be used within the user's sole to improve the load-bearing performance of the shoe and prevent buckling.

In the context of the present disclosure other examples and variations of the apparatus and methods described herein will be apparent to a person of skill in the art.

## Claims

1. An exoskeleton (100) for providing support to a human user, the exoskeleton (100) comprising a torso portion (102) and leg portion (106), the torso portion (102) and leg portion (106) coupled via a hip joint (104);
wherein the hip joint (104) comprises a torso arm (202) for detachably coupling to the torso portion (102) and leg arm (204) for detachably coupling to a leg portion (106), wherein the torso arm (202) and leg arm (204) are both coupled to a central portion (220) of the hip joint (104) via respective rotation limiting joints;
wherein the rotation limiting joint for the torso arm (202) has a first range of motion about the central portion (220), and the rotation limiting joint for the leg arm (204) has a second range of motion about the central portion (220), wherein the second range of motion is less than the first range of motion.

2. The exoskeleton (100) of claim 1 further comprising a buttock support arm coupled to a buttock support portion (122), the buttock support arm coupled to the hip joint (104) via a buttock rotation limiting joint;
wherein the buttock support arm has a third range of motion, for example wherein the torso arm (202), the leg arm (204) and the buttock support arm are configured to rotate about a common axis of the hip joint (104).

3. The exoskeleton (100) of claim 1 or 2 wherein the leg portion (106) is configured to detachably engage with a shoe worn by the user, for example via magnets.

4. The exoskeleton (100) of claim 3 wherein the leg portion (106) comprises an upper leg brace (108) and a lower leg brace (112), and wherein the leg portion (106) comprises an ankle joint (114) configured to permit the shoe engaged with the leg portion (106) to rotate relative to the lower leg brace (112), for example wherein the leg portion (106) is configured to detachably engage with the heel portion of a user's shoe, and wherein the ankle joint (114) is configured to be above a portion of the heel of the shoe proximate to the vamp of the shoe, and optionally wherein the upper leg brace (108) and the lower leg brace (112) each comprise at least one ratchet strap operable to fasten the upper brace (108) and/or the lower brace (112) around the user's anatomy.

5. The exoskeleton (100) of any of the previous claims wherein the torso portion (102) comprises a back brace configured to provide a clam shell for enclosing at least a portion of a user's torso, for example wherein the clam shell comprises a hinge portion comprising a flexible material capable of shear loading, configured to permit bending of the hinge portion, and optionally wherein the back brace comprises at least one ratchet strap.

6. The exoskeleton (100) of any of the previous claims wherein each of the torso portion (102) and leg portion (106) comprise a dismountable attachment point, wherein the torso arm (202) and the leg arm (204) are configured to engage with a corresponding dismountable attachment point to fasten the torso arm (202) with the torso portion (102) and the leg arm (204) with the leg portion (106) respectively.

7. The exoskeleton (100) of claim 6 wherein the dismountable attachment point comprises a locking fork (410), wherein the locking fork (410) comprises a lever arm cam lock mechanism configured to engage with a corresponding mating portion of the respective torso arm (202) or the leg arm (204), for example wherein the locking forks (410) are configured to operate in a locked configuration and a released configuration, and wherein the locking forks (410) are biased to remain in a locked configuration via magnets (408A).

8. The exoskeleton (100) of any preceding claim wherein the torso portion (102) comprises a hip shield (116), wherein the hip shield (116) is arranged on the opposite side of the exoskeleton (100) to the hip joint (104).

9. A hip joint (104) for coupling portions of an exoskeleton (100), the hip joint (104) comprising:
a first torso arm (202) coupled to a central portion (206) via a first rotation limiting joint having a first range of motion, wherein the first torso arm is configured to detachably couple to a torso portion of an exoskeleton;
a second leg arm (204) coupled to the central portion (206) via a second rotation limiting joint having a second range of motion, wherein the second leg arm is configured to detachably couple to a leg portion of said exoskeleton; and
wherein the second range of motion is less than the first range of motion.

10. The joint (104) of claim 9 further comprising a third arm coupled to the central portion (206) via a third rotation limiting joint having a third range of motion, for example wherein the first torso arm (202), second leg arm (204) and third arm are each configured to rotate about the central portion (206) via a common axis of rotation.

## Patentansprüche

1. Exoskelett (100) zum Bereitstellen einer Stütze für einen menschlichen Benutzer, wobei das Exoskelett (100) einen Torsoabschnitt (102) und einen Beinabschnitt (106) umfasst, wobei der Torsoabschnitt (102) und der Beinabschnitt (106) mittels eines Hüftgelenks (104) gekoppelt sind;
wobei das Hüftgelenk (104) einen Torso-Arm (202) zum lösbaren Koppeln an den Torsoabschnitt (102) und einen Bein-Arm (204) zum lösbaren Koppeln an einen Beinabschnitt (106) umfasst, wobei der Torso-Arm (202) und der Bein-Arm (204) beide mittels jeweiliger drehungsbegrenzender Gelenke an einen zentralen Abschnitt (220) des Hüftgelenks (104) gekoppelt sind;
wobei das drehungsbegrenzende Gelenk für den Torso-Arm (202) einen ersten Bewegungsbereich um den zentralen Abschnitt (220) aufweist und das drehungsbegrenzende Gelenk für den Bein-Arm (204) einen zweiten Bewegungsbereich um den zentralen Abschnitt (220) aufweist, wobei der zweite Bewegungsbereich kleiner als der erste Bewegungsbereich ist.

2. Exoskelett (100) nach Anspruch 1, ferner umfassend einen Gesäßstützarm, der an einen Gesäßstützabschnitt (122) gekoppelt ist, wobei der Gesäßstützarm mittels eines gesäßdrehungsbegrenzenden Gelenks an das Hüftgelenk (104) gekoppelt ist;
wobei der Gesäßstützarm einen dritten Bewegungsbereich aufweist, beispielsweise wobei der Torso-Arm (202), der Bein-Arm (204) und der Gesäßstützarm dazu konfiguriert sind, sich um eine gemeinsame Achse des Hüftgelenks (104) zu drehen.

3. Exoskelett (100) nach Anspruch 1 oder 2, wobei der Beinabschnitt (106) dazu konfiguriert ist, einen Schuh, der von dem Benutzer getragen wird, lösbar in Eingriff zu nehmen, beispielsweise mittels Magneten.

4. Exoskelett (100) nach Anspruch 3, wobei der Beinabschnitt (106) eine obere Beinorthese (108) und eine untere Beinorthese (112) umfasst, und wobei der Beinabschnitt (106) ein Knöchelgelenk (114) umfasst, das dazu konfiguriert ist, zuzulassen, dass sich der Schuh, der mit dem Beinabschnitt (106) im Eingriff steht, in Bezug auf die untere Beinorthese (112) dreht, beispielsweise wobei der Beinabschnitt (106) dazu konfiguriert ist, den Absatzabschnitt eines Schuhs eines Benutzers lösbar in Eingriff zu nehmen, und wobei das Knöchelgelenk (114) dazu konfiguriert ist, oberhalb eines Abschnitts des Absatzes des Schuhs nahe dem Blatt des Schuhs zu sein, und optional wobei die obere Beinorthese (108) und die untere Beinorthese (112) jeweils mindestens einen Spanngurt umfassen, der dazu bedienbar ist, die obere Orthese (108) und/oder die untere Orthese (112) um die Anatomie des Benutzers zu befestigen.

5. Exoskelett (100) nach einem der vorhergehenden Ansprüche, wobei der Torsoabschnitt (102) eine hintere Orthese umfasst, die dazu konfiguriert ist, eine Muschelschale zum Umschließen mindestens eines Abschnitts eines Torsos eines Benutzers bereitzustellen, beispielsweise wobei die Muschelschale einen Scharnierabschnitt umfasst, der ein flexibles Material umfasst, das zur Scherbelastung fähig ist, und dazu konfiguriert ist, ein Biegen des Scharnierabschnitts zuzulassen, und optional wobei die hintere Orthese mindestens einen Spanngurt umfasst.

6. Exoskelett (100) nach einem der vorhergehenden Ansprüche, wobei jeder von dem Torsoabschnitt (102) und dem Beinabschnitt (106) einen demontierbaren Anbringungspunkt umfasst, wobei der Torso-Arm (202) und der Bein-Arm (204) dazu konfiguriert sind, einen entsprechenden demontierbaren Anbringungspunkt in Eingriff zu nehmen, um den Torso-Arm (202) mit dem Torsoabschnitt (102) bzw. den Bein-Arm (204) mit dem Beinabschnitt (106) zu befestigen.

7. Exoskelett (100) nach Anspruch 6, wobei der demontierbare Anbringungspunkt eine Verriegelungsgabel (410) umfasst, wobei die Verriegelungsgabel (410) einen Hebelarm-Nockenverriegelungsmechanismus umfasst, der dazu konfiguriert ist, einen entsprechenden zusammenpassenden Abschnitt des jeweiligen Torso-Arms (202) oder des Bein-Arms (204) in Eingriff zu nehmen, beispielsweise wobei die Verriegelungsgabeln (410) dazu konfiguriert sind, in einer verriegelten Konfiguration und einer entriegelten Konfiguration zu arbeiten, und wobei die Verriegelungsgabeln (410) mittels Magneten (408A) vorgespannt sind, um in einer verriegelten Konfiguration zu bleiben.

8. Exoskelett (100) nach einem vorhergehenden Anspruch, wobei der Torsoabschnitt (102) einen Hüftschutz (116) umfasst, wobei der Hüftschutz (116) auf der entgegengesetzten Seite des Exoskeletts (100) zu dem Hüftgelenk (104) eingerichtet ist.

9. Hüftgelenk (104) zum Koppeln von Abschnitten eines Exoskeletts (100), wobei das Hüftgelenk (104) umfasst:
einen ersten Torso-Arm (202), der mittels eines ersten drehungsbegrenzenden Gelenks mit einem ersten Bewegungsbereich an einen zentralen Abschnitt (206) gekoppelt ist, wobei der erste Torso-Arm dazu konfiguriert ist, sich lösbar an einen Torsoabschnitt eines Exoskeletts zu koppeln;
einen zweiten Bein-Arm (204), der mittels eines zweiten drehungsbegrenzenden Gelenks mit einem zweiten Bewegungsbereich an den zentralen Abschnitt (206) gekoppelt ist, wobei der zweite Bein-Arm dazu konfiguriert ist, sich lösbar an einen Beinabschnitt des besagten Exoskeletts zu koppeln; und
wobei der zweite Bewegungsbereich kleiner als der erste Bewegungsbereich ist.

10. Gelenk (104) nach Anspruch 9, ferner umfassend einen dritten Arm, der mittels eines dritten drehungsbegrenzenden Gelenks mit einem dritten Bewegungsbereich an den zentralen Abschnitt (206) gekoppelt ist, beispielsweise wobei der erste Torso-Arm (202), der zweite Bein-Arm (204) und der dritte Arm jeweils dazu konfiguriert sind, sich mittels einer gemeinsamen Drehachse um den zentralen Abschnitt (206) zu drehen.

## Revendications

1. Exosquelette (100) destiné à fournir un support à un utilisateur humain, l'exosquelette (100) comprenant une partie torse (102) et une partie jambe (106), la partie torse (102) et la partie jambe (106) étant couplées via une articulation de hanche (104) ;
dans lequel l'articulation de hanche (104) comprend un bras de torse (202) destiné à se coupler de manière détachable à la partie torse (102) et un bras de jambe (204) destiné à se coupler de manière détachable à une partie jambe (106), dans lequel le bras de torse (202) et le bras de jambe (204) sont tous les deux couplés à une partie centrale (220) de l'articulation de hanche (104) via des articulations de limitation de rotation respectives ;
dans lequel l'articulation de limitation de rotation pour le bras de torse (202) a une première plage de mouvement autour de la partie centrale (220), et l'articulation de limitation de rotation pour le bras de jambe (204) a une deuxième plage de mouvement autour de la partie centrale (220), dans lequel la deuxième plage de mouvement est inférieure à la première plage de mouvement.

2. Exosquelette (100) selon la revendication 1 comprenant en outre un bras de support de fesse couplé à une partie support de fesse (122), le bras de support de fesse couplé à l'articulation de hanche (104) via une articulation de limitation de rotation de fesse ;
dans lequel le bras de support de fesse a une troisième plage de mouvement, par exemple dans lequel le bras de torse (202), le bras de jambe (204) et le bras de support de fesse sont configurés pour tourner autour d'un axe commun de l'articulation de hanche (104).

3. Exosquelette (100) selon la revendication 1 ou 2 dans lequel la partie jambe (106) est configurée pour entrer en prise de manière détachable avec une chaussure portée par l'utilisateur, par exemple via des aimants.

4. Exosquelette (100) selon la revendication 3 dans lequel la partie jambe (106) comprend une attelle de jambe supérieure (108) et une attelle de jambe inférieure (112), et dans lequel la partie jambe (106) comprend une articulation de cheville (114) configurée pour permettre à la chaussure entrée en prise avec la partie jambe (106) de tourner par rapport à l'attelle de jambe inférieure (112), par exemple dans lequel la partie jambe (106) est configurée pour entrer en prise de manière détachable avec la partie talon de la chaussure d'un utilisateur, et dans lequel l'articulation de cheville (114) est configurée pour être au-dessus d'une partie du talon de la chaussure à proximité de l'empeigne de la chaussure, et facultativement dans lequel l'attelle de jambe supérieure (108) et l'attelle de jambe inférieure (112) comprennent chacune au moins une sangle à cliquet capable de fonctionner pour fixer l'attelle supérieure (108) et/ou l'attelle inférieure (112) autour de l'anatomie de l'utilisateur.

5. Exosquelette (100) selon l'une quelconque des revendications précédentes dans lequel la partie torse (102) comprend une attelle dorsale configurée pour fournir une coque pour enfermer au moins une partie du torse d'un utilisateur, par exemple dans lequel la coque comprend une partie charnière comprenant un matériau flexible capable de supporter une charge de cisaillement, configurée pour permettre la flexion de la partie charnière, et facultativement dans lequel l'attelle dorsale comprend au moins une sangle à cliquet.

6. Exosquelette (100) selon l'une quelconque des revendications précédentes dans lequel chacune de la partie torse (102) et de la partie jambe (106) comprend un point d'attache démontable, dans lequel le bras de torse (202) et le bras de jambe (204) sont configurés pour entrer en prise avec un point d'attache démontable correspondant pour fixer le bras de torse (202) avec la partie torse (102) et le bras de jambe (204) avec la partie jambe (106) respectivement.

7. Exosquelette (100) selon la revendication 6 dans lequel le point d'attache démontable comprend une fourche de verrouillage (410), dans lequel la fourche de verrouillage (410) comprend un mécanisme de verrouillage à came de bras de levier configuré pour entrer en prise avec une partie d'accouplement correspondante du bras de torse respectif (202) ou du bras de jambe (204), par exemple dans lequel les fourches de verrouillage (410) sont configurées pour fonctionner dans une configuration verrouillée et une configuration libérée, et dans lequel les fourches de verrouillage (410) sont sollicitées pour rester dans une configuration verrouillée via des aimants (408A).

8. Exosquelette (100) selon l'une quelconque des revendications précédentes dans lequel la partie torse (102) comprend un bouclier de hanche (116), dans lequel le bouclier de hanche (116) est agencé sur le côté opposé de l'exosquelette (100) à l'articulation de hanche (104).

9. Articulation de hanche (104) destinée à coupler des parties d'un exosquelette (100), l'articulation de hanche (104) comprenant :
un premier bras de torse (202) couplé à une partie centrale (206) via une première articulation de limitation de rotation ayant une première plage de mouvement, dans laquelle le premier bras de torse est configuré pour se coupler de manière détachable à une partie torse d'un exosquelette ;
un deuxième bras de jambe (204) couplé à la partie centrale (206) via une deuxième articulation de limitation de rotation ayant une deuxième plage de mouvement, dans laquelle le deuxième bras de jambe est configuré pour se coupler de manière détachable à une partie jambe dudit exosquelette ; et
dans laquelle la deuxième plage de mouvement est inférieure à la première plage de mouvement.

10. Articulation (104) selon la revendication 9 comprenant en outre un troisième bras couplé à la partie centrale (206) via une troisième articulation de limitation de rotation ayant une troisième plage de mouvement, par exemple dans laquelle le premier bras de torse (202), le deuxième bras de jambe (204) et le troisième bras sont chacun configurés pour tourner autour de la partie centrale (206) via un axe de rotation commun.
